# EUROPÄISCHE PATENTSCHRIFT

(11) **EP 0 750 900 B1**
(45) Veröffentlichungstag und Bekanntmachung des Hinweises auf die Patenterteilung: **06.08.1997**
(21) Anmeldenummer: 96108135.3
(22) Anmeldetag: 22.05.1996
(51) Int. Cl.: A61K 7/06

(54) **Mittel zur Haarbehandlung**
Composition for hair treatment
Composition pour le traitement des cheveux

(30) Priorität: 29.06.1995 DE 19523651; 10.02.1996 DE 19604845
(43) Veröffentlichungstag der Anmeldung: 02.01.1997
(73) Patentinhaber: GOLDWELL GmbH, D-64297 Darmstadt (DE)
(72) Erfinder: Hinz, Sabine, 64319 Pfungstadt (DE); Heinz, Dieter, 65462 Gustavsburg (DE)

(56) Entgegenhaltungen:
- EP-A- 0 005 807
- EP-A- 0 287 876
- GB-A- 2 164 558
- DATABASE WPIL, AN 96-075 296, DERWENT PUBLICATIONS LTD., London; & JP-A-07 331 281 (ADJINOMOTO KK)
- G.A. NOWAK "Die kosmetischen PrUparate", 3. Auflage, Band 2, VERLAG F R CHEMISCHE INDUSTRIE H. ZIOLKOWSKY KG, Augsburg, 1984 Seiten 391-423 * Seiten 412-422 *
- H. JANISTYN "Die kosmetischen Grundstoffe", 3. Auflage, Band I, DR. ALFRED H THIG VERLAG, Heidelberg * Seiten 730,770,771 *

## Beschreibung

Die vorliegende Erfindung betrifft ein Mittel zur Haarbehandlung, insbesondere zur Behandlung von gespaltenen Haarspitzen, mit verbesserten Gebrauchseigenschaften.

Gespaltene Haarspitzen sind eine häufig auftretende Erscheinungsform geschädigten Haares, insbesondere nach starker Beanspruchung durch Dauerwellen, Sonneneinstrahlung oder sonstige Wärmebehandlung oder auch mechanische und Umwelteinflüsse.

Zur Behandlung gespaltener Haarenden oder gerissenen Haares wurden bereits verschiedene Präparate vorgeschlagen, insbesondere auf Basis höherviskoser Öle, wodurch zwar ein gewisser Klebeeffekt, aber auch ein unschönes, fettiges Aussehen des so behandelten Haares bewirkt wird.

Aus der EP-A 0 287 876 sind bereits haarregenerierende Zubereitungen bekannt, die Gemische aus 0,1 bis 8 Gew.-% Panthenol und 0,1 bis 8 Gew.-% eines Mono- oder Disaccharids enthalten. Fucose ist in diesem Zusammenhang nicht erwähnt.

Aus der EP-A 285 364 sind Mittel zur Behandlung gerissener Haare bzw. von Haaren mit gespaltenen Spitzen bekannt, die aus einem hochmolekularen Silikon, vorzugsweise gelöst in einem Öl oder niedrigsiedenden silikon, bestehen. Diese Zusammensetzungen sind ebenfalls bis zu einem gewissen Grad geeignet, gerissenes Haar oder gespaltene Haarspitzen zusammenzufügen, weisen jedoch aufgrund ihrer starken Hydrophobie ebenfalls den Nachteil auf, nach der Behandlung nicht aus dem Haar auswaschbar zu sein. Dadurch verleihen sie dem Haar nicht nur ein unästhetisches Aussehen, sondern beeinträchtigen auch etwaige nachfolgende Haarbehandlungsverfahren, insbesondere Dauerwellung und Haarfärbung.

Es bestand daher ein Bedürfnis nach Haarbehandlungsmitteln, die diese Nachteile nicht aufweisen.

Die vorliegende Erfindung löst diese Aufgabe durch ein Mittel zur Haarbehandlung, das nicht nur gespaltene Haarspitzen wieder schließt, sondern auch aus dem Haar gut auswaschbar ist und als wirksamen Bestandteil Fucose, vorzugsweise in einer Menge von mindestens 0,1 Gew.-%, berechnet auf die Gesamtzusammensetzung des Mittels, in Kombination mit Polyacrylamid enthält.

Der Obergehalt an Fucose wird vorwiegend durch die Kosten dieses Wirkstoffs bestimmt, er liegt vorzugsweise bei etwa 5 Gew.-% der Gesamtzusammensetzung des Mittels.

Der bevorzugte Bereich beträgt 0,25 bis 2,5, vorzugsweise bis etwa 1 Gew.-% des Mittels.

Obwohl natürlich die Mitverwendung von Silikon weiterhin möglich ist, ist dies durch den Einsatz von Fucose überflüssig geworden, so daß auch die mit dem Aufbringen von Silikonen auf das menschliche Haar verbundenen Kumulationseffekte vermieden werden.

Das erfindungsgemäße Mittel liegt vorzugsweise in Form eines opaken oder transparenten, viskosen Gels vor, obwohl prinzipiell natürlich auch andere Applikationsformen wie Lösungen, Emulsionen, Dispersionen, etc. möglich sind.

Aus Applikationsgründen ist jedoch ein höherviskoses Produkt klar zu bevorzugen.

Die erfindungsgemäß eingesetzte Fucose kann als reiner Zucker (d. h., D- und L-Fucose) oder in Form eines Saccharid-Gemisches vorliegen, wobei letzteres bevorzugt wird.

Ein geeignetes, mit Fucose angereichertes Polysaccharid-Gemisch ist beispielsweise das Handelsprodukt "Fucogel 1000®", das durch biotechnologische Fermentation eines pflanzlichen Ausgangsproduktes erhalten wird.

Es enthält neben Fucose insbesondere noch Galactose- und Galacturonsäure-Einheiten.

Ein bevorzugter zusätzlicher Bestandteil zur Wirkungssteigerung der erfindungsgemäßen Mittel, insbesondere der entsprechenden Gele, ist amorphes Siliciumdioxid, vorzugsweise in einer Menge von 0,5 bis 10, insbesondere 0,75 bis 7,5, besonders bevorzugt 1 bis 5 Gew.-% der Gesamtzusammensetzung.

Das amorphe Silicagel kann als solches oder in Verbindung mit organischen Substanzen zugesetzt werden.

Ein in diesem Zusammenhang einsetzbares Produkt wird unter dem Handelsnamen "Cosmacol PLG®" vertrieben und enthält neben dem amorphen Silica noch Di-C₁₂-C₁₆-alkyltartrate und Tri-C₁₂-C₁₆-alkylcitrate.

Der Anteil des erfindungsgemäßen zweiten Bestandteils, des Polyacrylamids, beträgt vorzugsweise etwa 0,2 bis etwa 4 Gew.-%, berechnet auf die Gesamtzusammensetzung des Mittels, insbesondere etwa 0,5 bis 2,5 Gew.-%.

Das Molgewicht des Polyacrylamids liegt dabei zwischen etwa 10 000 und 1 000 000, vorzugsweise etwa 25 000 bis 500 000, jedoch ist selbstverständlich auch die Mitverwendung höhermolekularer Produkte möglich.

Auch das Polyacrylamid kann als solches, beispielsweise als unter dem Handelsnamen "Reten 402®" vertriebenes Produkt, oder als Bestandteil eines Gemisches zugesetzt werden, beispielsweise mit langkettigen (Iso-)Paraffinen und Fettalkoholethoxylaten, z. B. Laureth-7.

Es wird hierzu auch auf das CTFA International Cosmetic Ingredient Dictionary, 4th Ed., S. 454, verwiesen.

Ein solchermaßen geeignetes Handelsprodukt ist beispielsweise unter dem Namen "Seppigel 305®" auf dem Markt.

Die Wirksamkeit eines Fucose enthaltenden Haarbehandlungsmittels läßt sich dadurch erhöhen, daß man diesem noch 1 bis 20 Gew.-%, berechnet auf die Gesamtzusammensetzung, mindestens eines Fettsäureesters der allgemeinen Formel 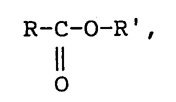 worin R einen gerad- oder verzweigtkettigen C₆-C₁₂-Alkylrest und R' einen gerad- oder verzweigtkettigen C₁₄-C₂₂-Alkylrest bedeuten, zusetzt.

Bevorzugte Fettsäureester sind solche, wie sie im natürlichen Bürzeldrüsenöl von Wasservögeln, insbesondere Enten ("Purcellinöl") enthalten sind, oder deren synthetische Nachstellungen, die unter der Bezeichnung "PCL" im Handel sind.

Besonders geeignete Ester sind dabei Cetyl-/Stearyloctanoat und Stearylheptanoat, vorzugsweise in einer Menge von etwa 1,5 bis etwa 15, insbesondere etwa 2,5 bis etwa 10 Gew.-% der Zusammensetzung.

Selbstverständlich können die erfindungsgemäßen Zusammensetzungen weitere in Haarpflegemitteln übliche Stoffe wie Feuchthaltemittel, Parfums, Weichmacher, Polymere, Öle, Fette, Emulgatoren, etc., soweit nötig und zweckmäßig, enthalten.

Insbesondere die Mitverwendung von Pflanzenölen, Pflanzenextrakten und α-Hydroxycarbonsäuren, d. h. Fruchtsäuren wie Weinsäure, Milchsäure, Citronensäure, Apfelsäure, Glykolsäure, Glyoxylsäure, Brenztraubensäure, und/oder Pyrrolidoncarbonsäure wird empfohlen.

Der pH-Wert der erfindungsgemäßen Mittel ist nicht kritisch und liegt zwischen etwa 4 und 8.

Die folgenden Beispiele illustrieren die Erfindung.

### Beispiel 1

### Haarspitzengel

| | |
|---|---|
| Fucogel 1000® (enthaltend 0,2 Gew.-% Fucose) | 70 (Gew.-%) |
| Seppigel 305® (enthaltend ∼ 40 % Polyacrylamid) | 2 |
| α-Hydroxycarbonsäure-Gemisch (Fruchtsäuren) | 1 |
| Wasser | 27 |

Diese erfindungsgemäße Zusammensetzung wurde in einem Vergleichsversuch auf 10 Haarsträhnen mit gespaltenen Haarspitzen aufgebracht. Dies geschah in der Weise, daß die Lösung mit Daumen und Zeigefinger vom Haarende zur Haarspitze hin appliziert wurde. Nach fünfminütiger Einwirkungszeit wurden die Haarsträhnen gewaschen und manuell sowie visuell unter dem Stereolichtmikroskop begutachtet.

Parallel dazu wurden 10 Haarsträhnen in gleicher Weise mit einem dem Stand der Technik entsprechenden Präparat folgender Zusammensetzung behandelt:

| | | |
|---|---|---|
| Nichtflüchtiges Dimethyl-polysiloxan | (Viskosität: 50.000 cSt bei 25°C) | 20 Gew.-% |
| Flüchtiges Dimethyl-polysiloxan | (Viskosität: 5 cSt bei 25°C) | 80 Gew.-% |

Bei der Auswertung zeigte sich, daß die mit den erfindungsgemäßen Produkten behandelten Haarspitzen vollständig zu einem einheitlichen Strang verbunden und keine Produktrückstände im Haar zurückgeblieben waren. Die mit dem bekannten Präparat behandelten Haarsträhnen zeigten eine ungleichmäßige Verklebung der Spitzen; das Präparat war darüber hinaus nicht vollständig aus dem Haar auswaschbar; das Haar wies einen fettigen Glanz auf.

In den folgenden Beispielen 2 und 3 werden Zusammensetzungen beschrieben, deren Eigenschaften dem im Beispiel 1 wiedergegebenen Produkt weitgehend entsprechen.

### Beispiel 2

### Haarspitzengel

| | |
|---|---|
| Fucogel 1000® (enthaltend 0,2 Gew.-% Fucose) | 70 Gew.-% |
| Seppigel 305® (enthaltend ∼ 40 Gew.-% Polyacrylamid) | 2 |
| Cosmacol PLG® (enthaltend ∼ 80 Gew.-% amorphes SiO₂) | 5 |
| Hydroxycarbonsäure-Gemisch (Fruchtsäuren) | 1 |
| Wasser | 22 |

### Beispiel 3

### Haarspitzengel

| | |
|---|---|
| Fucogel 1000® (enthaltend 0,2 Gew.-% Fucose) | 60,0 (Gew.-%) |
| Seppigel 305® (enthaltend ∼ 40 Gew.-% Polyacrylamid) | 2,0 |
| Pural MF 125® (Triglycerid-Öl, erhalten aus Lamnanthes alba) | 5,0 |
| Jaguar C-162® (Hydroxypropylguarhydroxypropyltrimoniumchlorid) | 0,5 |
| Hydroxycarbonsäure-Gemisch (Fruchtsäuren) | 1,0 |
| Wasser | 31,5 |

### Beispiel 4

### Haarspitzenfluid

| | |
|---|---|
| Fucogel 1000® (enthaltend 0,2 Gew.-% Fucose) | 20,0 (Gew.-%) |
| Panthenol | 0,1 |
| Seppigel 305® (enthaltend ∼ 40 % Polyacrylamid) | 3,0 |
| N-Laurylpyrrolidon | 0,2 |
| UV-Absorber (Parsol® MCS) | 0,5 |
| Cetyl-/Stearyloctanoat (PCL-liquid) | 4,0 |
| Isopropylmyristat | 1,0 |
| Parfum | 0,2 |
| Wasser | @ 100,0 |

Diese erfindungsgemäße Zusammensetzung wurde in einem Vergleichsversuch auf 10 Haarsträhnen mit gespaltenen Haarspitzen aufgebracht. Dies geschah in der Weise, daß die Lösung mit Daumen und Zeigefinger vom Haarende zur Haarspitze hin appliziert wurde. Nach fünfminütiger Einwirkungszeit wurden die Haarsträhnen gewaschen und manuell sowie unter dem stereolichtmikroskop begutachtet.

Parallel dazu wurden 10 Haarsträhnen in gleicher Weise mit einem dem Stand der Technik entsprechenden Präparat folgender Zusammensetzung behandelt:

| | | |
|---|---|---|
| Nichtflüchtiges Dimethyl-polysiloxan | (Viskosität: 50.000 cSt bei 25°C) | 20 Gew.-% |
| Flüchtiges Dimethyl-polysiloxan | (Viskosität: 5 cSt bei 25°C) | 80 Gew.-% |

Bei der Auswertung zeigte sich, daß die mit den erfindungsgemäßen Produkten behandelten Haarspitzen vollständig zu einem einheitlichen Strang verbunden und keine Produktrückstände im Haar zurückgeblieben waren. Die mit dem bekannten Präparat behandelten Haarsträhnen zeigten eine ungleichmäßige Verklebung der Spitzen; das Präparat war darüber hinaus nicht vollständig aus dem Haar auswaschbar, das Haar wies einen fettigen Glanz auf.

## Patentansprüche

1. Mittel zur Haarbehandlung, gekennzeichnet durch einen Gehalt an Fucose in Kombination mit Polyacrylamid.

2. Mittel nach Anspruch 1, dadurch gekennzeichnet, daß es mindestens 0,1 Gew.-% Fucose, berechnet auf die Gesamtzusammensetzung, enthält.

3. Mittel nach Anspruch 1 oder 2, dadurch gekennzeichnet, daß es mindestens 0,5 Gew.-%, berechnet aufdie Gesamtzusammensetzung, eines amorphen Siliciumdioxids enthält.

4. Mittel nach einem oder mehreren der Ansprüche 1 bis 3, dadurch gekennzeichnet, daß es in Form eines Gels vorliegt.

5. Haarbehandlungsmittel, enthaltend Fucose und 1 bis 20 Gew.-%, berechnet auf die Gesamtzusammensetzung, mindestens eines Fettsäureesters der allgemeinen Formel 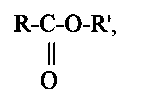 worin R einen gerad- oder verzweigtkettigen C₆-C₁₂-Alkylrest, und R' einen gerad- oder verzweigtkettigen C₁₂C₂₂-Alkylrest bedeuten, enthält.

6. Mittel nach Anspruch 5, dadurch gekennzeichnet, daß es 1,5 bis 15 Gew.-%, berechnet auf die Gesamtzusammensetzung, Cetyl-/Stearyloctanoat enthält.

7. Mittel nach Anspruch 5, dadurch gekennzeichnet, daß es 2,5 bis 10 Gew.-%, berechnet auf die Gesamtzusammensetzung, Stearylheptanoat enthält.

8. Verwendung einer Fucose enthaltenden Zusammensetzung zur Behandlung von gespaltenen Haarspitzen.

9. Verwendung einer eine Kombination aus Fucose und mindestens einem Fettsäureester der allgemeinen Formel 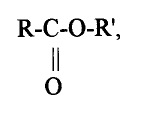 worin R einen gerad- oder verzweigtkettigen C₆-C₁₂-Alkylrest und R' einen gerad- oder verzweigkettigen C₁₄-C₂₂-Alkylrest bedeuten, enthaltenden Zusammensetzung zur Behandlung von gespaltenen Haarspitzen.

## Claims

1. Composition for the treatment of hair, containing fucose in combination with polyacrylamide.

2. Composition according to claim 1, containing at least 0.1% by wt., calculated to the total composition, fucose.

3. Composition according to claim 1 or 2, containing at least 0.5% by wt., calculated to the total composition, of amorphous silica.

4. Composition according to one or more of claims 1 to 3, being a gel.

5. Composition, containing fucose and 1% to 20% by wt., calculated to the total composition, of at least one fatty acid ester of the general formula 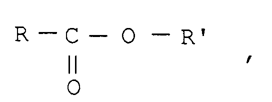 wherein R stands for a straight or a branched-chain C₆-C₁₂-alkyl group and R' is a straight or branched-chain C₁₂-C₂₂-alkyl group.

6. Composition according to claim 5, characterized in that it comprises 1.5% to 15% by wt.,calculated to the total composition, cetyl/stearyl octanoate, .

7. Composition according to claim 5, characterized in that it comprises 2.5% to 10% by wt., calculated to the total composition, stearyl heptanoate.

8. Use of a composition comprising fucose for the treatment of split hair ends.

9. Use of a combination of fucose and at least one fatty acid ester of the general formula 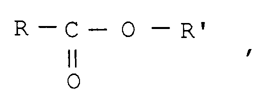 wherein R stands for a straight- or branched-chain C₆-C₁₂-alkyl group, and R' is a straight or a branched-chain C₁₄-C₂₂-alkyl group, for the treatment of split hair ends.

## Revendications

1. Composition pour le traitement des cheveux, contenant une combinaison de la fucose avec polyacrylamide.

2. Composition selon les revendication 1, contenant au moins 0.1% en poids de la fucose, calculé à la composition totale.

3. Composition selon les revendications 1 ou 2, contenant au moins 0.5% en poids, calcule à la composition totale, du silice amorphe.

4. Composition selon l'une quelconque des revendications 1 à 3, étant un gel.

5. Composition pour la traitement des cheveux, contenant fucose et 1% à 20% en poids de la composition totale d' au moins d'un ester d'acides gras de la formule générale 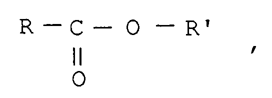 dons R est un groupe d'alkyle avec 6 à 12 atomes de carbone ramifié ou non-ramifié, et R' est un groupe d'alkyle avec 12 a 22 atomes de carbone, ramifié ou non-ramifié.

6. Composition selon la revendication 5, comprenant 1.5% à 15% en poids de la composition totale, d'octanoate de cétyle/stéaryle.

7. Composition selon la revendication 5, comprenant 2.5% à 10% en poids de la composition totale, du heptanoate de stéaryle.

8. Usage d'une composition comprenant de la fucose pour la traitement des pointes clivées des cheveux.

9. Usage d'une combinaison de la fucose et au moins d'un ester d'acides gras de la formule générale 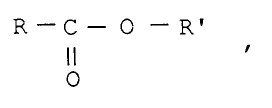 dans R est un groupe d'alkyl avec 6 à 12 atomes de carbone, ramifié ou non-ramifié, et R' est un groupe d'alkyle avec 12 à 22 atomes de carbone, ramifié ou non-ramifié, pour la traitement des pointes clivées des cheveux.
